# EUROPÄISCHE PATENTSCHRIFT

(11) **EP 2 559 444 B1**
(45) Veröffentlichungstag und Bekanntmachung des Hinweises auf die Patenterteilung: **01.06.2016**
(21) Anmeldenummer: 12179849.0
(22) Anmeldetag: 09.08.2012
(51) Int. Cl.: A61L 2/08, B65B 55/08, B67C 7/00, B29C 49/42

(54) **Verfahren und Vorrichtung zum Sterilisieren von Kunststoffbehältnissen mit Schutzvorrichtung**
Method and device for sterilising plastic containers with protective device
Procédé et dispositif de stérilisation de récipients en plastique avec dispositif de protection

(30) Priorität: 19.08.2011 DE 102011052862
(43) Veröffentlichungstag der Anmeldung: 20.02.2013
(73) Patentinhaber: Krones AG, 93073 Neutraubling (DE)
(72) Erfinder: Knott, Josef, 84069 Schierling (DE); Frankenberger, Günter, 93096 Köfering (DE); Scheuren, Hans, 55543 Bad Kreuznach (DE)
(74) Vertreter: Bittner, Bernhard

(56) Entgegenhaltungen:
- EP-A1- 2 161 202
- EP-A1- 2 491 955
- WO-A1-2011/067393
- WO-A2-2008/129397
- US-A1- 2011 012 032

## Beschreibung

Die vorliegende Erfindung bezieht sich auf eine Vorrichtung und ein Verfahren zum Sterilisieren von Behältnissen und insbesondere von Kunststoffbehältnissen. Aus dem Stand der Technik sind diverse Verfahren zum Sterilisieren von Behältnissen bekannt. So ist es bekannt, Behältnisse mit einem flüssigen und/oder gasförmigen Medium, wie beispielsweise H₂O₂ zu beaufschlagen. Daneben sind in jüngerer Zeit auch Vorrichtungen und Verfahren bekannt geworden, bei denen die Behältnisse mit Strahlung und/oder mit Ladungsträgern zum Zwecke der Sterilisation beaufschlagt werden. Dabei ist es möglich, einen Strahlfinger in das Innere der Behältnisse einzuführen und dieses Behältnis von innen mit Ladungsträgem und insbesondere mit Elektronen zu bestrahlen. Diese Strahlfinger müssen dabei relativ filigran bzw. dünnwandig ausgeführt werden, um einerseits ein Einführen durch die Mündung in das Innere der Behältnisse zu erreichen und um andererseits auch die notwendigen hohen Elektronenbeschleunigungen zu ermöglichen. Weiterhin weisen derartige Vorrichtungen oftmals noch Kühlvorrichtungen zum Kühlen eines Austrittsfensters für die Ladungsträger auf.

Dies führt dazu, dass derartige Sterilisationsvorrichtungen sehr empfindlich beispielsweise gegenüber Verschmutzungen oder auch aggressiven Reinigungsmitteln, aber insbesondere gegenüber Stößen, sind. In der Praxis kommt es daher immer wieder zu Ausfällen und Störungen infolge von beispielsweise gegen diese Strahlfinger gerichteten Stößen, insbesondere während Reinigungs- oder Wartungsarbeiten.

Aus der US 2011/0012032 A1 ist eine Vorrichtung zum Sterilisieren einer Flasche bekannt. Die Vorrichtung weist dabei einen bewegbaren Elektronenstrahlemitter, eine Vielzahl von Greifern, einen oder mehrere stationäre Elektronenstrahlemitter, zum Sterilisieren der Außenwand der Flasche und eine Steuerung auf, welche mit der Vielzahl an Greifern und dem bewegbaren Elektronenstrahlemitter verbunden ist.

Der vorliegenden Erfindung liegt daher die Aufgabe zugrunde, eine Vorrichtung und ein Verfahren zu schaffen, welche einen verbesserten Schutz der Vorrichtung zum Sterilisieren der Behältnisse ermöglichen. Diese Aufgabe wird erfindungsgemäß durch die Gegenstände der unabhängigen Ansprüche erreicht. Vorteilhafte Ausführungsformen und Weiterbildungen sind Gegenstand der Unteransprüche.

Eine erfindungsgemäße Vorrichtung zum Sterilisieren von Behältnissen weist einen beweglichen Träger auf, an dem eine Vielzahl von Sterilisationseinrichtungen angeordnet ist, wobei diese Sterilisationseinrichtungen jeweils eine Strahlungsquelle bzw. Strahlungseinrichtung und/oder eine Ladungsträgerquelle aufweisen, sowie einen stangenartigen Körper, der derart ausgestaltet ist, dass er durch eine Mündung in einen Innenraum der Behältnisse einführbar ist. Weiterhin ist an dem stangenartigen Körper ein Austrittsfenster vorgesehen, durch welches die Ladungsträger austreten können. Weiterhin ist eine Vielzahl von Halteeinrichtungen zum Halten der Behältnisse vorgesehen, wobei die Halteeinrichtungen bezüglich der stangenartigen Körper in einer Längsrichtung der stangenartigen Körper bewegbar sind.

Erfindungsgemäß weist die Vorrichtung eine Schutzeinrichtung auf, welche bewirkt, dass die stangenartigen Körper in einem vorgegebenen Betriebsmodus der Vorrichtung wenigstens teilweise innerhalb von Schutzkörpern angeordnet sind.

Die Erfindung wird im Folgenden unter Bezugnahme auf Ladungsträgerstrahler und insbesondere Elektronenstrahler beschrieben. Aufgrund der hohen Empfindlichkeit derartiger Ladungsträgerstrahler ist die Erfindung auch hierfür besonders vorteilhaft. Es wird jedoch darauf hingewiesen, dass die Erfindung auch auf andere Strahlungseinrichtungen wie insbesondere UV - Lampen, Röntgenstrahler oder radioaktive Strahler, welche insbesondere in die Behältnisse eingeführt werden können, anwendbar ist.

Es wird daher vorgeschlagen, dass in wenigstens einem Modus der Vorrichtung besondere Schutzvorkehrungen zum Schutz insbesondere der stangenartigen Körper, aber auch beispielsweise des Austrittsfensters vorgenommen werden. Insbesondere soll daher ein Schutz der Strahlfinger während eines Reinigungs- und Sterilisationsprozesses der Anlage vorgesehen sein. Daher wird hier vorgeschlagen, während dieser Vorgänge beispielsweise entsprechende Behälter über die zu schützenden stangenartigen Körper zu bewegen bzw. zu fahren.

Unter einem Modus wird insbesondere ein Betriebsmodus der Vorrichtung verstanden, wobei während dieses Betriebsmodus nicht notwendigerweise Behältnisse sterilisiert werden. So wird etwa auch die Reparatur der Anlage oder die Reinigung als ein derartiger Modus verstanden. Bevorzugt handelt es sich jedoch um einen Modus, bei dem wenigstens ein Antrieb der Vorrichtung aktivierbar ist, etwa ein Antrieb, der eine Hub- oder Senkbewegung der Halteeinrichtung bewirken kann. Auch können im Rahmen eines derartigen Modus Montagen oder Demontagen von Elementen der Vorrichtung vorgenommen werden, wobei jedoch die Montage d.h. der (Erst-)aufbau der gesamten Anlage insbesondere nicht als ein Betriebsmodus im Sinne der Erfindung angesehen wird.

Wie oben erwähnt, sind die stangenartigen Körper gegenüber den Halteeinrichtungen bzw. gegenüber den von diesen Halteeinrichtungen gehaltenen Behältnissen bewegbar. Dabei ist es möglich, dass die Halteinrichtungen in der Längsrichtung der Behältnisse bewegbar sind, es wäre jedoch auch möglich, dass die stangenartigen Körper selbst bewegt werden, oder auch, dass sowohl die stangenartigen Körper, als auch die Halteeinrichtungen bewegt werden. Bei den Ladungsträgern handelt es sich insbesondere um Elektronen.

Bei einem weiteren vorteilhaften Verfahren werden sämtliche stangenartige Körper in dem besagten Modus jeweils in die Schutzkörper eingefahren. Vorteilhaft umgeben die Schutzkörper zumindest das Austrittsfenster vollständig und besonders bevorzugt sind sie nach unten hin beziehungsweise zum Ende der stangenartigen Körper hin abgeschlossen.

Es ist jedoch auch möglich und bevorzugt, dass die Schutzkörper einen großen Teil des stangenartigen Körpers und bevorzugt den gesamten stangenartigen Körper in dessen Umfangsrichtung umgeben. Bevorzugt umgeben die Schutzkörper den stangenartigen Körper auf wenigstens 30% seiner Länge, bevorzugt auf wenigstens 50% seiner Länge, bevorzugt auf wenigstens 70% seiner Länge und besonders bevorzugt auf wenigstens 90% seiner Länge.

Bei einer weiteren vorteilhaften Ausführungsform sind die stangenartigen Körper an einem Träger angeordnet. Unter der Schutzeinrichtung wird im Folgenden die gesamte Einrichtung einschließlich der Schutzkörper und etwaiger Bewegungsmechanismen für die Schutzkörper verstanden. Vorteilhaft ist in dem besagten Modus an jedem stangenartigen Körper genau ein Schutzkörper, beispielsweise eine Schutzkappe angeordnet. Vorteilhaft fällt, wenn der stangenartige Körper in den Schutzkörper eingefahren ist, eine Symmetrieachse des stangenartigen Körpers mit einer Symmetrieachse des Schutzkörpers zusammen.

Insbesondere werden bevorzugt in dem besagten Modus auch diejenigen stangenartigen Körper mit Schutzkörpern versehen bzw. in diese eingefahren, die sich in dem besagten Modus an Positionen befinden, an denen im Arbeitsbetrieb keine Behältnisse angeordnet sind. Bei derartigen Positionen kann es sich beispielsweise um die Positionen zwischen einem Auslaufstem und einem Einlaufstern der Sterilisationsvorrichtung handeln.

Bei einer weiteren vorteilhaften Ausführungsform weist die Schutzeinrichtung einen Bewegungsmechanismus zum Bewegen der Schutzkörper in der Längsrichtung der stangenartigen Körper auf, der bewirkt, dass die stangenartigen Körper wenigstens teilweise in die Schutzkörper in dem vorgegeben Modus eingefahren sind. Vorteilhaft sind, wie oben erwähnt, sämtliche stangenartige Körper jeweils teilweise und bevorzugt gleichartig in die Schutzkörper eingefahren. Dabei kann es sich wiederum um einen Bewegungsmechanismus handeln, der die Schutzkörper in der Längsrichtung der Behältnisse bewegt und sie beispielsweise über die stangenartigen Körper schiebt. Es wäre jedoch auch denkbar, dass der Bewegungsmechanismus die stangenartigen Körper selbst bewegt bzw. in die Schutzkörper einfährt.

Bei einer weiteren vorteilhaften Ausführungsform handelt es sich bei dem Träger um einen drehbaren Träger. Bei dieser Ausführungsform ist daher die Vorrichtung als Rundläufermaschine ausgebildet.

Bei einer weiteren vorteilhaften Ausführungsform sind die Schutzkörper in dem vorgegebenen Betriebsmodus berührungslos gegenüber den stangenartigen Körpern angeordnet. Auf diese Weise kann erreicht werden, dass auch durch den Einführvorgang der stangenartigen Körper in die Schutzkörper keine Beschädigung der stangenartigen Körper auftritt.

Bei einer weiteren vorteilhaften Ausführungsform weist die Vorrichtung eine Reinigungseinrichtung zum Reinigen der Vorrichtung auf. Diese Reinigungseinrichtung kann dabei beispielsweise eine Vielzahl von Strahldüsen aufweisen, welche die Vorrichtung oder Teile der Vorrichtung mit einem Reinigungsmedium beaufschlagen. So könnten beispielsweise stationär angeordnete Reinigungsdüsen angeordnet sein und die einzelnen Reinigungseinrichtungen oder Sterilisationseinrichtungen können sich diesen Düsen gegenüber bewegen, um so gereinigt zu werden.

Daneben kann die Vorrichtung auch eine Sterilisationseinrichtung zum Sterilisieren der Vorrichtung selbst aufweisen. Auch die Sterilisation kann dabei mit Reinigungsmedien erfolgen, es wäre jedoch auch denkbar, dass die Sterilisation selbst wiederum durch Strahlung, wie UV-Strahlung oder auch Elektronenstrahlung, erfolgt.

Bei einer weiteren vorteilhaften Ausführungsform weist jede Sterilisationseinrichtung wenigstens eine elektromotorische Antriebseinrichtung zum Bewegen der Greifelemente bezüglich der stangenartigen Körper und/oder zum Bewegen der Schutzkörper gegenüber den stangenartigen Körpern in der Längsrichtung der stangenartigen Körper auf. Im Stand der Technik werden zum Bewegen derartiger Strahlfinger üblicherweise Kurvenrollen bzw. stationäre Kurven eingesetzt.

Im Falle der erfindungsgemäßen Vorrichtung ist es jedoch gewünscht, beispielsweise in einem Reinigungsbetrieb mehrere und bevorzugt sämtliche Strahlfinger - und insbesondere unabhängig von der Drehposition des Trägers - in ihre Schutzkörper einzufahren. In diesem Falle ist daher in diesem Reinigungsmodus eine einheitliche Bewegung bzw. Stellung aller Schutzkörper vorteilhaft. Daher wird vorgeschlagen, die Bewegungen der einzelnen Schutzkörper gegenüber den Strahlfingern voneinander zu entkoppeln. Mit anderen Worten erlauben insbesondere die elektromotorischen Antriebe (z.B. Linearmotoren und/oder Servomotoren) eine unabhängige Bewegung der einzelnen Greifelemente bzw. auch der Schutzkörper gegenüber den stangenartigen Körpern. Als Antriebe kämen jedoch auch pneumatische und/oder hydraulische Antriebe in Betracht.

Bei einer weiteren vorteilhaften Ausführungsform bewirkt die Schutzeinrichtung, dass die zu sterilisierenden Behältnisse als Schutzkörper dienen. Bei dieser Ausführungsform ist vorgesehen, dass keine zusätzlichen Schutzkörper vorhanden sind, sondern diejenigen Behältnisse, welche im Arbeitsbetrieb sterilisiert werden, auch als Schutzkörper dienen. Diese Behältnisse sind relativ gut geeignet, da die stangenartigen Körper ohnedies in diese eingefahren werden müssen. Dazu ist es möglich, dass beispielsweise in dem Reinigungsmodus die Behältnisse, d.h. Flaschen oder Vorformlinge, über die stangenartigen Körper bzw. die Strahlfinger gefahren werden. Vorteilhaft ist eine Strahlungsemission bzw. Ladungsträgeremission während dieser Zelt unterbrochen.

Bei einer weiteren vorteilhaften Ausführungsform weist die Vorrichtung wenigstens eine Sta-bilisierungseinrichtung auf, um die Schutzkörper gegenüber den stangenartigen Körpern zu fixieren bzw. zu sterilisieren. So kann beispielsweise ein Anschlag vorgesehen sein, an den die Behältnisse angelegt werden. Auf diese Weise kann erreicht werden, dass auch ein etwas heftigeres Anschlagen der Schutzkörper nicht zu einer Beschädigung der Strahlfinger führt.

Es wäre jedoch auch möglich, dass eine Halterung vorgesehen ist, welche die Behältnisse beispielsweise von unten her stabilisiert.

Die vorliegende Erfindung ist weiterhin auf ein Verfahren zum Betreiben einer Vorrichtung zum Sterilisieren von Behältnissen und insbesondere von Kunststoffbehältnissen gerichtet. Dabei werden die zu sterilisierenden Behältnisse jeweils gehalten und mittels eines beweglichen Trägers entlang einer vorgegebenen Bahn transportiert. Dabei ist eine Vielzahl von an dem Träger angeordneten Sterilisationseinrichtungen vorgesehen, die jeweils stangenartige Körper aufweisen, welche durch eine Mündung in diese Behältnisse eingeführt werden und eine Innenwandung der Behältnisse mit Ladungsträgern und/oder mit Strahlung beaufschlagen. Dabei treten diese Ladungsträger und/oder die Strahlung durch ein Austrittsfenster der stangenartigen Körper aus und eine Relativbewegung der Behältnisse findet in einer Längsrichtung der stangenartigen Körper gegenüber den stangenartigen Körpern statt, um die stangenartigen Körper in die Behältnisse einzuführen.

Erfindungsgemäß werden in einem weiteren Modus der Vorrichtung die stangenartigen Körper jeweils in diese zumindest teilweise umgebende Schutzkörper eingeführt und dieses Einführen erfolgt ebenfalls durch eine Relativbewegung zwischen den Schutzkörpern und den stangenartigen Körpern in einer Längsrichtung der stangenartigen Körper.

Wie oben erwähnt kann es sich bei der Strahlung um eine Ladungsträgerstrahlung handeln, jedoch auch um andere Strahlungsarten wie etwa UV - Strahlung. Im Falle von UV - Strahlung kann das Austrittsfenster auch erheblich größer gewählt werden als im Falle eines Elektronenstrahlers und beispielsweise die UV - Lichtquelle vollumfänglich umgeben. Insbesondere erfolgt dieses Einführen der stangenartigen Körper in die Schutzkörper, um eine Reinigung durchzuführen. Vorteilhaft werden hierbei die stangenartigen Körper in die zu sterilisierenden Behältnisse eingefahren. Es wäre jedoch auch möglich, zusätzliche Körper, wie beispielsweise zusätzliche Schutzkappen, vorzusehen.

Bei einem weiteren vorteilhaften Verfahren werden während des besagten Modus, der beispielsweise ein Reinigungsmodus sein kann, sämtliche stangenartige Körper insbesondere im Wesentlichen gleich weit in die Schutzkörper gefahren. In diesem Falle werden also auch Schutzkörper an Stellen angeordnet, an denen diese im Arbeitsbetrieb nicht vorhanden sind, wie beispielsweise in Umfangsrichtung zwischen einem Auslaufstern und einem Einlaufstern des Trägers.

Vorteilhaft wird damit jede Sterilisationseinrichtung mit einem Schutzkörper bestückt.

Vorteilhaft wird in einem Verfahrensschritt die Vorrichtung mit Schutzkörpern bestückt, wobei besonders bevorzugt jede Sterilisationseinheit mit einem Schutzkörper bestückt wird. Dies erfolgt aber insbesondere automatisch. So wäre es möglich, dass vor einem Reinigungsbetrieb zunächst jede Sterilisationseinheit mit einem Behältnis bestückt wird und der jeweilige Strahlfinger bzw. stangenartige Körper in dieses Behältnis eingefahren wird.

In einem bevorzugten Verfahren werden in dem besagten Modus zwei aufeinanderfolgende Reinigungs- und/oder Sterilisationsverfahren durchgeführt.

Bei einem Verfahrensablauf kann dabei, wie folgt, vorgegangen werden:
Nach dem Ende des Produktionsprozesses wird jeder stangenartige Körper vorzugsweise mittels des jeweiligen Hubmechanismus in einen Schutzkörper eingefahren. Bei diesem Schutzkörper kann es sich um eine spezielle Schutzform, um das Behältnis oder auch um einen Vorformling handeln. Im Unterschied zum Produktionsbetrieb sind dabei jedoch alle stangenartigen Körper in einem Schutzkörper angeordnet.

Anschließend wird die Anlagentechnik heruntergefahren, beispielsweise vorübergehend stillgesetzt. In einem weiteren Verfahrensschritt kann die Anlage geöffnet werden, um eine Reinigung vorzunehmen. Im Anschluss hieran findet bevorzugt eine händische Reinigung der Anlage statt. In einem weiteren Schritt kann die Anlage geschlossen werden (insbesondere mit einem Sicherheitsprotokoll). Schließlich kann noch eine automatische Sterilisation der Anlage durchgeführt werden und schließlich der Produktionsprozess wieder beginnen.

Bei einer weiteren vorteilhaften Ausführungsform weist daher die Vorrichtung auch einen Reinraum auf, innerhalb dessen die zu sterilisierenden Behältnisse geführt werden. Dieser Reinraum kann dabei vorteilhaft den Transportpfad der Behältnisse kanalartig umgeben. Dies bedeutet, dass die Sterilisation der Behältnisse auch unter sterilen Bedingungen durchgeführt wird.

Weiterhin wäre es auch denkbar, dass wenigstens eine Wandung des oben erwähnten drehbaren Trägers auch gleichzeitig eine Wandung dieses Reinraumes ausbildet.

Weitere Vorteile und Ausführungsformen ergeben sich aus den beigefügten Figuren:

Darin zeigen:
- Fig. 1: eine schematische Darstellung einer erfindungsgemäßen Vorrichtung; und
- Fig. 2a,b: zwei Darstellungen zur Veranschaulichung der Erfindung.

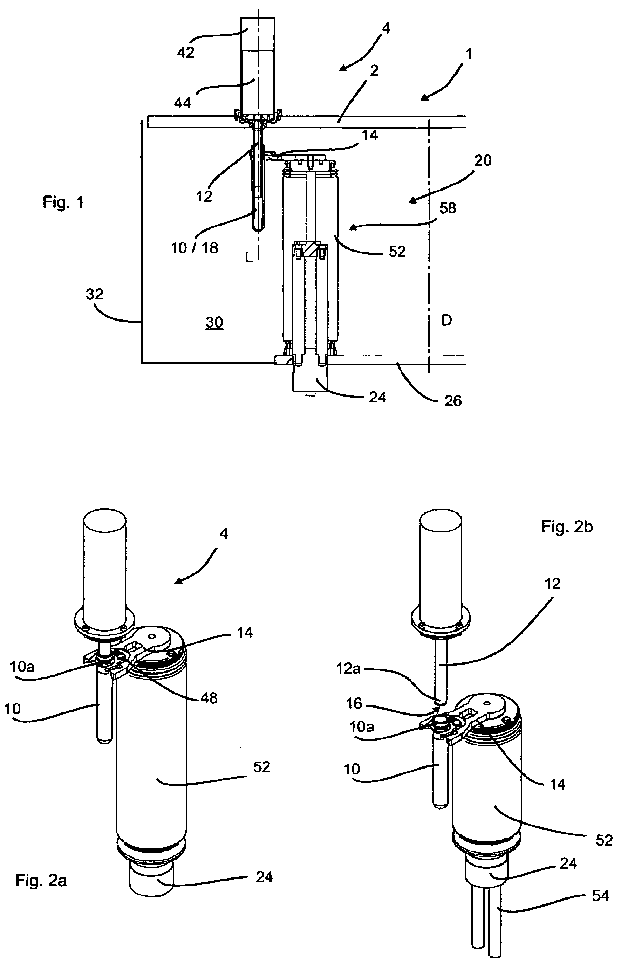

Fig. 1 zeigt eine teilweise schematische Darstellung einer erfindungsgemäßen Vorrichtung 1 zum Sterilisieren von Behältnissen 10. Bei der in Fig. 1 gezeigten Ausführungsform handelt es sich bei den Behältnissen um Kunststoffvorformlinge 10, deren Innenwandungen hier sterilisiert werden sollen. An die erfindungsgemäße Vorrichtung 1 können sich im Rahmen einer Behältnisbehandlungsanlage weitere Einrichtungen anschließen, wie beispielsweise eine Streckblasmaschine, welche die Kunststoffvorformlinge zu Behältnissen expandiert, eine Heizeinrichtung zum Erwärmen der Kunststoffvorformlinge und dergleichen. Es wäre jedoch auch möglich, die erfindungsgemäße Vorrichtung zum Sterilisieren von Behältnissen und insbesondere Kunststoffbehältnissen zu verwenden.

An einem drehbaren Träger 2 ist eine Vielzahl von in ihrer Gesamtheit mit 4 bezeichneten Sterilisationseinrichtungen angeordnet, wobei jedoch nur eine dieser Sterilisationseinrichtungen 4 gezeigt ist. Diese Sterilisationseinrichtungen 4 bewegen sich gemeinsam mit den zu sterilisierenden Kunststoffbehältnissen.

Die Sterilisationseinrichtungen 4 weisen jeweils eine Ladungsträgerquelle 42, bei der es sich insbesondere um eine Elektronenquelle handelt, auf. Das Bezugszeichen 44 kennzeichnet eine Beschleunigungseinrichtung, welche die Ladungsträger bzw. Elektronen in Richtung eines stangenförmigen Körpers 12 und auch durch diese hindurch beschleunigt. Am unteren Ende dieses stangenartigen Körpers 12 ist ein Austrittsfenster (nicht gezeigt) angeordnet, durch welches hindurch die beschleunigten Elektronen austreten können.

Daneben weisen die Sterilisationseinrichtungen bevorzugt noch Ablenkeinrichtungen (nicht gezeigt) auf, welche die Ladungsträger auch auf eine seitliche Innenwandung der Kunststoffbehältnisse richten bzw. lenken.

Das Bezugszeichen 14 kennzeichnet eine Halteeinrichtung, wie etwa eine Greifklammer, die zum Halten der Kunststoffvorformlinge 10 während des Sterilisationsvorgangs dient. Diese Halteeinrichtung 14 ist dabei in der Längsrichtung L der Kunststoffbehältnisse 10 beweglich, um so die stangenartigen Körper 12 in das Innere der Kunststoffbehältnisse 10 durch eine Mündung der Kunststoffbehältnisse 10 hindurch einzuführen. Eine entsprechende Antriebseinrichtung 24, welche hier als Elektromotor, insbesondere als Linearmotor ausgeführt ist, bewirkt die Bewegung der Halteeinrichtungen 14 relativ zu den stangenartigen Körpern 12.

Diese Antriebseinrichtungen 24 sind dabei ebenfalls an einem Träger 26 angeordnet, wobei dieser Träger 26 sich - ebenso wie der Träger 2 - um die Drehachse D dreht.
Das Bezugszeichen 30 kennzeichnet einen Reinraum, innerhalb dessen die Kunststoffbehältnisse sterilisiert werden. Dieser Raum wird hier durch den Träger 2, den Träger 26 und zwei weitere Wandungen (nicht gezeigt) abgegrenzt. Dabei kann auch eine insbesondere stehende Aussenwandung 32 vorgesehen sein, welche in Figur 1 nur schematisch dargestellt ist und welche den Reinraum 30 nach außen hin abgrenzt. Vorteilhaft ist diese Aussenwandung 32 stehend angeordnet, wohingegen sich die übrigen den Reinraum 30 begrenzenden Wandungen bewegen bzw. um die Drehachse D drehen.

Bei einer weiteren vorteilhaften Ausführungsform ist wenigstens eine Dichtungseinrichtung (nicht gezeigt) vorgesehen, welche diese bezüglich einander beweglichen Wände gegeneinander abdichtet. Ein Beispiel für eine derartige Dichtungseinrichtung wäre ein sog. Wasserschloss. Es wäre jedoch auch möglich, dass die ganze Vorrichtung 1 innerhalb eines Reinraums angeordnet ist. Auch wäre es denkbar, dass die Vorrichtung nicht innerhalb eines Reinraums angeordnet ist, etwa, wenn lediglich eine Vorsterilisierung der Kunststoffbehältnisse vorgenommen werden soll.

Bei der in den Figuren dargestellten Ausführungsform werden die zu sterilisierenden Kunststoffbehältnisse auch gleichzeitig als Schutzkörper 18 verwendet. So können - etwa im Rahmen eines Reinigungs- und/oder Sterilisationsbetriebs- die stangenartigen Körper 12 in die Kunststoffvorformlinge eingefahren werden. Bevorzugt weisen jedoch allgemein die Schutzkörper einen Hohlraum auf, in den die stangenartigen Körper einführbar sind. Vorteilhaft weisen auch die Schutzkörper mit Ausnahme derjenigen Öffnung, über die der jeweilige stangenartige Körper 12 eingeführt wird, keine weiteren Öffnungen auf. Es wäre jedoch auch denkbar, dass die Schutzkörper an ihrem unteren Ende eine Öffnung aufweisen, über welche ein Reinigungsmedium (mit dem beispielsweise die stangenartigen Körper 12 beaufschlagt werden, abgeführt werden kann. Das Bezugszeichen 20 kennzeichnet in seiner Gesamtheit die Schutzeinrichtung zum Schützen der stangenförmigen Körper 12.

Die Fig. 2a und 2b zeigen zwei Darstellungen einer erfindungsgemäßen Vorrichtung 1, wobei bei der in Fig. 2a gezeigten Situation die stangenartigen Körper 12 in die Kunststoffvorformlinge 10 eingefahren sind und so eine Reinigung der Anlage ohne die Gefahr einer Beschädigung der stangenartigen Körper 12 möglich ist. Man erkennt hier wiederum die Mündung 10a der Behältnisse, über welche die stangenartigen Körper in das Behältnis 10 bzw. den Schutzkörper 18 eingeführt werden.

Das Bezugszeichen 16 bezieht sich auf ein Austrittsfenster, über welches die Elektronen aus dem stangenartigen Körper 12 bzw. Strahlfinger 12 austreten können. Dieses Austrittsfenster ist in einem Endabschnitt 12a des stangenartigen Körpers 10 angeordnet. Vorteilhaft ist auch eine Kühleinrichtung zum Kühlen dieses Austrittsfensters vorgesehen, wobei hierzu ein entsprechendes Kühlmedium beispielsweise zwischen einem Außengehäuse des stangenartigen Körpers und einem innerhalb dieses stangenartigen Gehäuses befindlichen Innengehäuse geleitet werden kann.

Das Bezugszeichen 48 kennzeichnet grob schematisch eine Stabilisierungseinrichtung. Bei der in Fig. 2a gezeigten Situation kann diese Stabilisierungseinrichtung 48 an eine entsprechend komplementär ausgebildete, beispielsweise an dem Träger 2 ausgebildete Stabilisierungseinrichtung (nicht gezeigt) herangeführt werden, um so den Schutzkörper 18 gegenüber dem Träger zu stabilisieren. Allgemein kann diese Stabilisierungseinrichtung 48 derart ausgeführt sein, dass sie eine Stabilisierung gegenüber dem Träger 2 oder allgemein gegenüber einem Element bewirkt, an dem auch der stangenartige Körper 12 fest angeordnet ist.

Auf diese Weise kann während des Reinigungsbetriebs eine Relativbewegung zwischen dem Schutzkörper 14 und dem stangenartigen Körper 12 verhindert werden. Es wäre jedoch auch möglich, dass die Greifeinrichtung 14 gegenüber ihrer Bewegungseinrichtung stabilisiert wird. Auch könnten zusätzliche Stabilisierungseinrichtungen (nicht gezeigt) vorgesehen sein, welche das Behältnis an einem Grundkörper unterhalb der Mündung abstützen, wie etwa ein zweites Greifelement, welches in der Längsrichtung L unterhalb der Greifeinrichtung 14 angeordnet ist. Auf diese Weise werden die Behältnisse in ihrer Längsrichtung an zwei unterschiedlichen Positionen gehalten, wodurch eine erhöhte Stabilisierung des Behältnisses 10 erreicht wird.

Die Antriebseinrichtung 24, welche im Arbeitsbetrieb dazu dient, um die stangenartigen Körper in die Kunststoffbehältnisse 10 einzufahren, dient im Reinigungsbetrieb auch dazu, um die stangenartigen Körper 12 in deren Schutzkörper 18 einzufahren. Zu diesem Zweck wäre es möglich, sämtliche stangenartigen Körper 12 der jeweiligen Sterilisationseinrichtungen in die Kunststoffvorformlinge bzw. Kunststoffbehältnisse einzufahren und bevorzugt in eine obere Position, wie in Fig. 2a gezeigt, zu bewegen.

Bevorzugt weist jede Sterilisationseinrichtung 4 auch eine derartige Antriebseinrichtung 24 auf, wobei diese Antriebseinrichtungen vorteilhaft unabhängig voneinander steuerbar sind. Vorteilhaft sind auch die Halte- bzw. Greifeinrichtungen 14 und insbesondere zwischen einem geschlossenen Zustand, in dem sie die Behältnisse 10 halten, und einem geöffneten Zustand, in dem die Behältnisse 10 freigegeben werden, steuerbar.

Um den oben erwähnten Reinigungsmodus einzuleiten ist es möglich, dass jede dieser Greifeinrichtungen mit einem als Schutzkörper 18 wirkenden Kunststoffbehältnis 10 besetzt wird.

Dies könnte auch über eine entsprechende Steuerung eines Auslaufsterns erfolgen, der im Arbeitsbetrieb die Kunststoffbehältnisse von der Vorrichtung abführt. Nach dem Durchführen einer Reinigung können die einzelnen Greifeinrichtungen 14 mit den daran angeordneten Kunststoffbehältnissen 10/18 wieder abgesenkt werden und die jeweiligen Kunststoffbehältnisse 10 können aus der Vorrichtung ausgefahren werden. Vorteilhaft werden die Behältnisse ausgeschleust bzw. entsorgt, wobei ein derartiges Ausschleusen vorteilhaft automatisch erfolgt.

Auch wäre es möglich, dass spezielle Schutzkörper verwendet werden, welche insbesondere zu Reinigungszwecken in die Vorrichtung eingefahren werden und welche bevorzugt nach der Reinigung wiederverwendet werden können. Vorteilhaft sind derartige Schutzkörper so gestaltet, dass sie von den Greifeinrichtungen 14 gegriffen werden können. Derartige Schutzkörper könnten dabei so dimensioniert sein, dass die Gefahr einer Beschädigung der einzelnen stangenartigen Körper weiter verringert wird, etwa einen Durchmesser aufweisen, der deutlich größer ist als der Durchmesser der stangenartigen Körper.

Das Bezugszeichen 52 kennzeichnet eine Abdeckeinrichtung, welche den Bewegungsmechanismus zum Heben und Senken der Halteinrichtung abdeckt. Bei dieser Abdeckeinrichtung kann es sich beispielsweise um einen Faltenbalg handeln. Diese Abdeckeinrichtung ist dabei vorteilhaft innerhalb des Reinraumes 30 angeordnet bzw. bildet selbst eine Grenze des Reinraumes 30 aus. Auch kann ein Teil der Abdeckung ein Gehäuse sein, an dem bevorzugt ein zusätzlicher Faltenbalg zur Ermöglichung einer Hub- und Senkbewegung angeordnet ist.

Innerhalb der Abdeckeinrichtung ist ein von der Antriebseinrichtung 24, (und damit bevorzugt von außerhalb des Reinraumes) angetriebener Hebe- und Senkmechanismus 58 zum Heben und Senken der Halteeinrichtung vorgesehen.

Dieser Hebe- und Senkmechanismus kann dabei Haltestangen 54 aufweisen, die von der Antriebseinrichtung 24 bewegt werden.

Auch wäre es möglich, dass die Halte- bzw. Greifeinrichtungen für die Kunststoffbehältnisse 10 auch zum Greifen anderer Schutzkörper geeignet sind, etwa in dem die Greifeinrichtungen auch die Aufnahme von Objekten mit Querschnitten erlauben, welche größer sind als die Querschnitte der zu sterilisierenden Behältnisse 10.

Es wäre jedoch auch möglich, dass an der Vorrichtung bereits Schutzkörper angeordnet sind, die - beispielsweise über einen separaten Hubmechanismus - über die stangenartigen Körper gefahren werden. Auch könnten die Schutzkörper als die stangenartigen Körper nur teilweise umgebende Schilde ausgebildet sein. Die erfindungsgemäße Schutzvorrichtung ist ebenso geeignet zum Schutz von beispielsweise stangenartigen UV-Lampen, die zur Behältnisbehandlung in Behältnisse eingefahren werden können.

### Bezugszeichenliste

- 1: Vorrichtung
- 2: Träger
- 4: Sterilisationseinrichtung
- 10: Behältnis
- 10a: Mündung des Behältnisses
- 12: stangenartiger Körper
- 12a: Endabschnitt des stangenartigen Körpers
- 14: Halteeinrichtung
- 16: Austrittsfenster
- 18: Schutzkörper
- 20: Schutzeinrichtung
- 24: Antriebseinrichtung
- 26: Träger
- 30: Reinraum
- 32: Wandung
- 42: Ladungsträgerquelle
- 44: Beschleunigungseinrichtung
- 48: Stabilisierungseinrichtung
- 52: Abdeckeinrichtung
- 54: Hubstange
- 58: Hebe- und Senkmechanismus
- L: Längsrichtung
- D: Drehachse

## Patentansprüche

1. Verfahren zum Betreiben einer Vorrichtung (1) zum Sterilisieren von Behältnissen (10) und insbesondere von Kunststoffbehältnissen, wobei die zu sterilisierenden Behältnisse (10) jeweils gehalten werden und mittels eines beweglichen Trägers (2) entlang einer vorgegebenen Bahn transportiert werden und wobei eine Vielzahl von an dem Träger (2) angeordneten Sterilisationseinrichtungen (4) vorgesehen ist, die jeweils stangenartige Körper (12) aufweisen, welche durch eine Mündung der Behältnisse (10) in diese Behältnisse (10) eingeführt werden und eine Innenwandung der Behältnisse (10) mit Ladungsträgern beaufschlagen, wobei diese Ladungsträger durch ein Austrittsfenster der stangenartigen Körper (12) austreten und wobei eine Relativbewegung der Behältnisse (10) in einer Längsrichtung der stangenartigen Körper (12) gegenüber den stangenartigen Körpern (12) stattfindet, um die stangenartigen Körper (12) in die Behältnisse (10) einzuführen, wobei dies ein erster Modus der Vorrichtung ist
**dadurch gekennzeichnet, dass**
in einem weiteren Modus der Vorrichtung (1) die stangenartigen Körper (12) jeweils in diese zumindest teilweise umgebende Schutzkörper (18) eingeführt werden und dieses Einführen ebenfalls durch eine Relativbewegung zwischen den Schutzkörpern (18) und den stangenartigen Körpern (12) in einer Längsrichtung (L) der stangenartigen Körper (12) erfolgt.

2. Verfahren nach Anspruch 1,
**dadurch gekennzeichnet, dass**
sämtliche stangenartigen Körper (12) in diesem Modus in die Schutzkörper (18) eingefahren werden.

3. Verfahren nach wenigstens einem der vorangegangenen Ansprüche,
**dadurch gekennzeichnet, dass**
in dem Modus zwei aufeinanderfolgende Reinigungs- und/oder Sterilisationsvorgänge durchgeführt werden.

4. Verfahren nach zumindest einem der vorhergehenden Ansprüche,
**dadurch gekennzeichnet, dass**
die Schutzeinrichtung (20) einen Bewegungsmechanismus zum Bewegen der Schutzkörper (18) in der Längsrichtung (L) der stangenartigen Körper (12) aufweist, der bewirkt, dass in dem vorgegebenen Modus die stangenartigen Körper (12) wenigstens teilweise in die Schutzkörper (18) eingefahren sind.

5. Verfahren nach wenigstens einem der vorangegangenen Ansprüche,
**dadurch gekennzeichnet, dass**
der Träger ein drehbarer Träger ist.

6. Verfahren nach wenigstens einem der vorangegangenen Ansprüche,
**dadurch gekennzeichnet, dass**
die Schutzkörper in dem vorgegebenen Betriebsmodus berührungslos gegenüber den stangenartigen Körpern (12) angeordnet sind.

7. Verfahren nach wenigstens einem der vorangegangenen Ansprüche,
**dadurch gekennzeichnet, dass**
die Vorrichtung (1) eine Reinigungseinrichtung zum Reinigen der Vorrichtung (1) aufweist.

8. Verfahren nach Anspruch 1,
**dadurch gekennzeichnet, dass**
jede Sterilisationseinrichtung (4) wenigstens eine elektromotorische Antriebseinrichtung (24) zum Bewegen der Halteeinrichtungen (14) bezüglich der stangenartigen Körper (12) und/oder zum Bewegen der Schutzkörper (18) gegenüber den stangenartigen Körpern (12) in der Längsrichtung der stangenartigen Körper (12) aufweist.

9. Verfahren nach wenigstens einem der vorangegangenen Ansprüche,
**dadurch gekennzeichnet, dass**
die zu sterilisierenden Behältnisse (10) als Schutzkörper (18) verwendbar sind.

10. Verfahren nach wenigstens einem der vorangegangenen Ansprüche,
**dadurch gekennzeichnet, dass**
die Vorrichtung (1) wenigstens eine Stabilisierungseinrichtung aufweist, umdie Schutzkörper (18) gegenüber den stangenartigen Körpern (12) zu fixieren.

11. Vorrichtung (1) zum Sterilisieren von Behältnissen, mit einem beweglichen Träger (2), an dem eine Vielzahl von Sterilisationseinrichtungen (4) angeordnet ist, wobei diese Sterilisationseinrichtungen jeweils eine Ladungsträgerquelle (42) aufweisen, sowie einen stangenartigen Körper (12) der derart ausgestaltet ist, dass er durch eine Mündung (10a) in einen Innenraum der Behältnisse (10) einführbar ist, mit einem an dem stangenartigen Körper (12) vorgesehenen Austrittsfenster, durch welches die Ladungsträger austreten können, mit einer Vielzahl von Halteeinrichtungen (14) zum Halten der Behältnisse, wobei die Halteeinrichtungen (14) bezüglich der stangenartigen Körper (12) in einer Längsrichtung (L) der stangenartigen Körper (12) bewegbar sind,
wobei die Vorrichtung (1) eine Schutzeinrichtung (20) aufweist, welche bewirkt, dass die stangenartigen Körper (12) in einem vorgegebenen Modus der Vorrichtung (1) wenigstens teilweise innerhalb von Schutzkörpern (18) angeordnet sind,
**dadurch gekennzeichnet, dass**
die Vorrichtung (1) zur Durchführung des Verfahrens gemäß zumindest einem der vorhergehenden Ansprüche ausgebildet ist.

## Claims

1. A method for operating an apparatus (1) for sterilising containers (10) and in particular plastics material containers, wherein the containers to be sterilized (10) are respectively held and are transported by means of a movable carrier (2) along a pre-defined path and wherein a plurality of sterilization means (4) arranged at the carrier (2) are provided which each have rod-like bodies (12), which are introduced into said containers (10) through a mouth of the containers (10) and act upon an inner wall of the containers (10) with charge carriers, wherein these charge carries exit through an exit window of the rod-like bodies (12) and wherein a relative movement of the containers (10) takes place in a longitudinal direction of the rod-like bodies (12) with respect to the rod-like bodies (12), in order to introduce the rod-like bodies (12) into the containers (10), wherein this is a first mode of the apparatus, **characterised in that**
in a further mode of the apparatus (1) the rod-like bodies (12) are respectively introduced into protective bodies (18) which at least partly surround the latter and that this introduction takes likewise place by means of a relative movement between the protective bodies (18) and the rod-like bodies (12) in a longitudinal direction (L) of the rod-like bodies (12).

2. Method according to claim 1,
**characterised in that**
all rod-like bodies (12) are inserted into the protective bodies (18) in this mode.

3. Method according to at least one of the preceding claims,
**characterised in that**
in the mode two subsequent cleaning and/or sterilization processes are carried out.

4. Method according to at least one of the preceding claims, **characterised in that** the protection device (20) includes a movement mechanism for moving the protective bodies (18) in the longitudinal direction (L) of the rod-like bodies (12), which protection device causes the rod-like bodies (12) to be at least partially inserted into the protective bodies (18) in the specified mode.

5. Method as claimed in at least one of the preceding claims, **characterised in that** the carrier is a rotatable carrier.

6. Method as claimed in at least one of the preceding claims, **characterised in that** in the specified mode of operation, the protective bodies are arranged in a contact-free manner relative to the rod-like bodies (12).

7. Method as claimed in at least one of the preceding claims, **characterised in that** the apparatus (1) includes a cleaning unit for cleaning the apparatus (1).

8. Method as claimed in claim 1, **characterised in that** each sterilisation unit (4) includes at least one electric motor drive unit (24) for moving the holding units (14) relative to the rod-like bodies (12) and/or for moving the protective bodies (18) relative to the rod-like bodies (12) in the longitudinal direction of the rod-like bodies (12).

9. Method as claimed in at least one of the preceding claims, **characterised in that** the containers (10) to be sterilised can serve as protective bodies (18).

10. Method as claimed in at least one of the preceding claims, **characterised in that** the apparatus (1) has at least one stabilisation unit in order to fix the protective bodies (18) relative to the rod-like bodies (12).

11. Apparatus (1) for sterilizing containers, with a movable carrier (2) at which a plurality of sterilization means (4) are arranged, wherein these sterilization means each have a charge carrier source (42) and a rod-like body (12) which is designed in such a manner that it can be introduced into the interior of the containers (10) through a mouth (10a) with an outlet window provided at the rod-like body (12) through which the charge carriers can exit, with a plurality of holding means (14) for holding the containers, wherein the holding means (14) are movable with respect to the rod-like bodies (12) in a longitudinal direction (L) of the rod-like bodies (12),
wherein the apparatus (1) has a protection device (20) which effects that the rod-like bodies (12) are arranged in a specified mode of the apparatus (1) at least partly inside protective bodfes (18),
**characterised in that**
the apparatus (1) is designed for carrying out the method according to at least one of the preceding claims.

## Revendications

1. Procédé permettant de faire fonctionner un dispositif (1) de stérilisation de contenants (10) et en particulier de contenants en matière plastique, les contenants (10) à stériliser étant retenus chacun et étant transportés au moyen d'un support (2) mobile le long d'un trajet prédéfini, et une pluralité de dispositifs de stérilisation (4) disposés sur le support (2) comprenant chacun des corps en forme de barre (12), lesquels sont introduits dans ces contenants (10) par une embouchure desdits contenants (10) et soumettent une paroi intérieure des contenants (10) à l'effet de porteurs de charge, ces porteurs de charge sortant par une fenêtre de sortie des corps en forme de barre (12) et les contenants (10) effectuant un déplacement relatif par rapport aux corps en forme de barre (12) dans une direction longitudinale des corps en forme de barre (12), afin d'introduire les corps en forme de barre (12) dans les contenants (10), ceci représentant un premier mode du dispositif,
**caractérisé en ce que**
dans un autre mode du dispositif (1), les corps en forme de barre (12) sont introduits chacun dans des corps de protection (18) entourant ces derniers au moins en partie, et grâce à un déplacement relatif entre les corps de protection (18) et les corps en forme de barre (12) selon une direction longitudinale (L) des corps en forme de barre (12).

2. Procédé selon la revendication 1,
**caractérisé en ce que**
dans ce mode, tous les corps en forme de barre (12) sont insérés dans les corps de protection (18).

3. Procédé selon au moins l'une quelconque des revendications précédentes,
**caractérisé en ce que**
dans ce mode, deux procédures de nettoyage et/ou de stérilisation successives sont mises en oeuvre.

4. Procédé selon au moins l'une quelconque des revendications précédentes,
**caractérisé en ce que**
le dispositif de protection (20) comprend un mécanisme de déplacement permettant de déplacer les corps de protection (18) dans la direction longitudinale (L) des corps en forme de barre (12), ce qui permet, dans le mode prédéfini, aux corps en forme de barre (12) d'être insérés au moins en partie dans les corps de protection (18).

5. Procédé selon au moins l'une quelconque des revendications précédentes,
**caractérisé en ce que**
le support est un support rotatif.

6. Procédé selon au moins l'une quelconque des revendications précédentes,
**caractérisé en ce que**
dans le mode de fonctionnement prédéfini, les corps de protection sont disposés sans contact par rapport aux corps en forme de barre (12).

7. Procédé selon au moins l'une quelconque des revendications précédentes,
**caractérisé en ce que**
le dispositif (1) comprend un système de nettoyage permettant de nettoyer le dispositif (1).

8. Procédé selon la revendication 1,
**caractérisé en ce que**
chaque dispositif de stérilisation (4) comprend au moins un dispositif d'entraînement électromoteur (24) permettant de déplacer les dispositifs de retenue (14) par rapport aux corps en forme de barre (12) et/ou de déplacer les corps de protection (18) par rapport aux corps en forme de barre (12) dans la direction longitudinale des corps en forme de barre (12).

9. Procédé selon au moins l'une quelconque des revendications précédentes,
**caractérisé en ce que**
les contenants (10) à stériliser peuvent être utilisés comme corps de protection (18).

10. Procédé selon au moins l'une quelconque des revendications précédentes,
**caractérisé en ce que**
le dispositif (1) comprend au moins un dispositif de stabilisation afin de fixer les corps de protection (18) par rapport aux corps en forme de barre (12).

11. Dispositif (1) de stérilisation de contenants, comprenant un support (2) mobile sur lequel une pluralité de dispositifs de stérilisation (4) sont disposés, ces dispositifs de stérilisation comprenant chacun une source de porteurs de charge (42), et un corps en forme de barre (12) qui est conçu de telle manière qu'il peut être introduit par une embouchure (10a) dans un espace intérieur des contenants (10), comprenant une fenêtre de sortie ménagée sur le corps en forme de barre (12) et par laquelle les porteurs de charge peuvent sortir, comprenant une pluralité de dispositifs de retenue (14) permettant de retenir les contenants, les dispositifs de retenue (14) étant mobiles par rapport aux corps en forme de barre (12) dans une direction longitudinale (L) des corps en forme de barre (12),
le dispositif (1) comprenant un dispositif de protection (20), lequel a pour effet que dans un mode prédéfini du dispositif (1), les corps en forme de barre (12) sont disposés au moins en partie à l'intérieur des corps de protection (18),
**caractérisé en ce que**
le dispositif (1) est conçu pour mettre en oeuvre le procédé selon au moins l'une quelconque des revendications précédentes.
